# EUROPÄISCHE PATENTSCHRIFT

(11) **EP 0 641 387 B1**
(45) Veröffentlichungstag und Bekanntmachung des Hinweises auf die Patenterteilung: **24.06.1998**
(21) Anmeldenummer: 93911727.1
(22) Anmeldetag: 28.04.1993
(51) Int. Cl.: C12P 7/62, C08G 63/90

(54) **VERFAHREN ZUR GEWINNUNG VON FARBSTOFFFREIEN POLYHYDROXYALKANOATEN**
PROCESS FOR PREPARING DYE-FREE POLYHYDROXYALKANOATES
PROCEDE D'OBTENTION DE POLYHYDROXYALCANOATES EXEMPTS DE COLORANTS

(30) Priorität: 14.05.1992 DE 4215860; 14.05.1992 DE 4215861; 14.05.1992 DE 4215862
(43) Veröffentlichungstag der Anmeldung: 08.03.1995
(73) Patentinhaber: Buna Sow Leuna Olefinverbund GmbH, 06258 Schkopau (DE)
(72) Erfinder: LEHMANN, Olaf, D-4090 Halle (DE); MAYER, Torsten, D-4070 Halle (DE); RAPTHEL, Inno, D-4090 Halle (DE); RAUCHSTEIN, Klaus-Dieter, D-4090 Halle (DE); RUNKEL, Dietmar, D-4200 Merseburg (DE); SCHAFFER, Jürgen, D-4090 Halle (DE)
(86) Internationale Anmeldenummer: DE9300372
(87) Internationale Veröffentlichungsnummer: WO9323554

(56) Entgegenhaltungen:
- EP-A- 0 355 307
- DD-A- 229 428
- DE-A- 1 720 325
- Plaste und Kautschuk, Jg. 36, Heft 5, 1989, S. 148

## Beschreibung

Die Erfindung betrifft ein Verfahren zur Gewinnung von farbstofffreien Polyhydroxyalkanoaten (PHA), welche als Speicherstoffe bei der Kultivierung von Bakterienbiomassen unter definierten Bedingungen in deren Zellen synthetisiert werden.

Unter definierten Fermentationsbedingungen synthetisieren verschiedene Mikroorganismen polymere β-Hydroxyalkansäuren zum Beispiel Polyhydroxybuttersäure (PHB) oder Copolymere Polyhydroxybuttersäure/Polyhydroxyvaleriansäure sowie auch andere Polyhydroxycarbonsäuren. Daneben entstehen in den Zellen auch immer Lipide und Farbstoffe, welche bei einer weiteren Verarbeitung der gewonnenen PHA als Verunreinigung störend wirken. Es sind Verfahren bekannt, bei denen Lipide und Farbstoffe durch niedere Alkohole oder Aceton (EP 15 123, EP 58 480, EP 124 309) entfernt werden. Bei einigen carotinoiden Farbstoffen führen diese Lösungsverfahren nicht zur gewünschten Farbreinheit, d. h. zu einem weißen Polymeren.

So ist nach EP 0355 307 A 2 zur Gewinnung von in 3-Hydroxybuttersäureeinheiten enthaltenen Polyestern oder Copolyestern durch Extraktion bekannt, Di- oder Tricarbonsäureester, Gemische von Dicarbonsäureester oder Butyrolacton einzusetzen. Dabei wird ausdrücklich darauf verwiesen, daß unter dem Butyrolacton ein γ-Butyrolacton zu verstehen ist.

Zur Extraktion eines Polyhydroxyalkanoates aus einem Mikroorganismusstamm Methylobacterium rhodesianum mit einem hohen Weißheitsgrad ist ein γ-Butyrolacton jedoch nicht zu verwenden.

Zur Gewinnung einer Poly-β-hydroxybuttersäure aus einer getrockneten Bakterienbiomasse wird ein Verfahren unter Verwendung von Essigsäureanhydrid als Extraktionsmittel vorgeschlagen (DD 229 428 A1).Bei diesem Verfahren wird jedoch die Zielstellung verfolgt, ohne Einsatz eines zusätzlich Fällmittels eine Poly-β-hydroxybuttersäure in fester Konsistenz zu erhalten. Auch hier weist bei der Verwendung eines Mikroorganismusstammes, welcher nebenbei carotinoide Farbstoffe produziert, die ausgefällte PHB nicht die erforderliche Qualität hinsichtlich des Weißheitsgrades auf.
In der Literatur,,Plaste und Kautschuk", 1989 (Jg. 36), Heft 5, S 148 wird unter anderem darauf verwiesen, daß Essigsäure (Eisessig) ein erfolgreiches Lösungsmittel für PHB darstellt.
Eine andere Möglichkeit wird durch das Umfällen des Polymeren mit einem PHA-Löser (DD 276 304) erreicht. Dabei ist einerseits mit einem Kettenlängenabbau zu rechnen, und andererseits wird durch die Anzahl und/oder Verschiedenheit der verwendeten Lösungs- und Fällmittel eine Aufarbeitung der PHA erschwert. Es ist auch ein höherer Restgehalt an Lösungs- oder Fällmitteln in den PHA zu erwarten.

Der Erfindung liegt die Aufgabe zugrunde, die im Prozeß der Kultivierung der Mikroorganismen und der Akkumulation von PHA in den Zellen gebildeten Farbstoffe aus den PHA zu entfernen.

Bei dem Verfahren zur Gewinnung von farbstofffreien PHA aus Bakterienbiomassen unter Anwendung der Verfahrensschritte Trocknung der feuchten Bakterienbiomasse, Extraktion der PHA mit Essigsäure und Ausfällen der im Extraktionsmittel gelösten PHA wird die Aufgabe erfindungsgemäß dadurch gelöst, daß die getrocknete Bakterienbiomasse vor der Extraktion einer zusätzlichen Temperaturbehandlung unterzogen wird. Dazu wird die getrocknete Bakterienbiomasse zwischen 10 Minuten und 120 Minuten einer Temperatur ausgesetzt, welche im Bereich zwischen 80 °C und der Temperatur der unteren Grenze des Schmelzbereiches der Polyhydroxyalkanoate liegt. Während dieser Temperatureinwirkung wird der in der Bakterienbiomasse enthaltene Farbstoff in eine solche Form überführt, die keinen störenden Einfluß auf die weitere Verarbeitung des Polymeren mehr ausübt. Im sich daran anschließenden Extraktionsprozeß werden dem Extraktionsmittel Essigsäure 1 bis 10 Volumenanteile in % Essigsäureanhydrid oder β-Butyrolacton, bezogen auf das Gesamtvolumen des Extraktionsmittels, zugesetzt.
Die Extraktion wird unter bekannten Bedingungen durchgeführt. Die sich der Extraktion anschließende Verfahrensstufe des Ausfällens der PHA aus dem Extraktionsmittel unter Anwendung bekannter Verfahrensbedingungen ergibt hellere PHA als bei bekannten Verfahrensweisen.

Das dem Extraktionsmittel zugesetzte Essigsäureanhydrid oder β-Butyrolacton und die Farbstoffe verbleiben im Extraktionsmittel.

Überraschenderweise wurde nach der Aufarbeitung der PHA mit dem erfindungsgemäßen Extraktionsmittelgemisch aus Essigsäure und Essigsäureanhydrid ein Produkt erhalten, dessen Helligkeit über der einer unbehandelten PHA lag, d. h., deren Farbstoffanteil sich verringert hat, im Vergleich zu einer Extraktion in der bekannten Weise mit einer reinen Essigsäure.

Obwohl die Extraktionsmittel Essigsäureanhydrid (oder β-Butyrolacton) eine größere Helligkeit der gewonnenen PHB ergeben, war nicht zu erwarten, daß der Zusatz einer geringen Menge, erfindungsgemäß zwischen 1 und 10 Vol.-%, Essigsäureanhydrid zum Extraktionsmittel Essigsäure eine wesentliche Steigerung der Helligkeit des Produktes im Gegensatz zur Verwendung dieses Produktes in reiner Form ergibt.

Es wird durch die Verwendung des Extraktionsmittels Essigsäure unter Zusatz von Essigsäureanhydrid oder β-Butyrolacton in geringer Menge ein sehr hoher Helligkeitsgrad der PHA erreicht.

Vorteilhaft bei der Verfahrensweise ist die Vermeidung des Einsatzes zusätzlicher Lösungsmittel für eine Vorextraktion der getrockneten Bakterienbiomasse, um den Farbstoffanteil vor der Extraktion der PHA aus der Bakterienbiomasse zu entfernen sowie der Erhalt eines Polymeren mit ausreichend hoher Kettenlänge durch Wegfall zusätzlicher Stufen der Umfällung der PHA.

Die Erfindung wird an folgenden Beispielen näher erläutert:

### Vergleichsbeispiel:

Sprühgetrocknete Bakterienbiomasse (Helligkeit L nach HUNTER Lab = 64,5) wird in einem Verhältnis 1 10 bei Siedetemperatur des Extraktionsmittels Essigsäure extrahiert und wie bekannt behandelt. Die gewonnene Polyhydroxybuttersäure (PHB) wurde mit L = 75,3 vermessen.

Die Erfindung wird an weiteren Beispielen näher erläutert:

### Beispiel 1

Die sprühgetrocknete Bakterienbiomasse wurde vor der Extraktion im Trockenschrank einer Temperaturbehandlung unterzogen. Die untere Grenze des Schmelzbereiches der Polyhydroxybuttersäure betrug 160 °C. Danach erfolgte eine Extraktion der behandelten Bakterienbiomasse im Verhältnis 1 : 10 mit Essigsäure, welcher in einem Fall 1 Vol.-%, und in einem anderen Fall 10 Vol.-% Essigsäureanhydrid bezogen auf das Gesamtvolumen des Extraktionsmittels zugesetzt wurden. Es ergaben sich folgende Helligkeiten L (nach HUNTER) für die PHB:

| Anteil Essigsäureanhydrid in Vol.-% zum Extraktionsmittel | 1 | | | | 10 | | | |
|---|---|---|---|---|---|---|---|---|
| Behandlungszeit in min | 10 | 10 | 120 | 120 | 10 | 10 | 120 | 12,0 |
| | | | | | | | | |
| Behandlungstemperatur in °C | 80 | 160 | 80 | 160 | 80 | 160 | 80 | 160 |
| | | | | | | | | |
| Helligkeit L | 91,9 | 92,0 | 92,0 | 92,2 | 92,2 | 92,8 | 92,2 | 93,2 |

### Beispiel 2

Entsprechend dem 1. Beispiel wurde die sprühgetrocknete Bakterienbiomasse vor der Extraktion einer Temperaturbehandlung unterzogen. Dem Extraktionsmittel Essigsäure wurde jedoch β-Butyrolacton von 1 Vol.-% bzw. 10 Vol.-% bezogen auf das Gesamtvolumen des Extraktionsmittels zugesetzt. Es ergaben sich folgende Helligkeiten L (nach HUNTER) für die PHB:

| Anteil β-Butyrolacton in Vol.-% zum Extraktionsmittel | 1 | | | | 10 | | | |
|---|---|---|---|---|---|---|---|---|
| Behandlungszeit in min | 10 | 10 | 120 | 120 | 10 | 10 | 120 | 120 |
| | | | | | | | | |
| Behandlungstemperatur in °C | 80 | 160 | 80 | 160 | 80 | 160 | 80 | 160 |
| | | | | | | | | |
| Helligkeit L | 80,8 | 82,0 | 81,8 | 82,4 | 82,3 | 86,7 | 87,7 | 90,3 |

## Patentansprüche

1. Verfahren zur Gewinnung von farbstofffreien Polyhydroxyalkanoaten aus Bakterienbiomasse, vorzugsweise aus der Bakterienbiomasse der Mikroorganismenkultur Methylobacterium rhodesianum IMET 11401 - DSM 8166 MB 126, mit den Verfahrensstufen
Trocknung der feuchten Biomasse,
Extraktion der getrockneten Biomasse mit Essigsäure und
Ausfällen der Polyhydroxyalkanoate
gekennzeichnet dadurch, daß die getrocknete Bakterienbiomasse in einem Zeitraum von 10 bis 120 Minuten einer Temperatur zwischen 80 °C und der unteren Grenze des Schmelzbereiches der Polyhydroxyalkanoate ausgesetzt und das Extraktionsmittel 1 bis 10 Volumenanteile in % Essigsäureanhydrid oder β-Butyrolacton, bezogen auf das Gesamtvolumen des Extraktionsmittels, enthält.

## Claims

1. A process to make pigment-free polyhydroxy alkanoates from bacterial biomass, preferably from the bacterial biomass of the microorganism culture of Methylobacterium rhodesianum IMET 11401 - DSM 8166 MB 126, with the process stages of
drying of the moist biomass
extraction of the dried biomass by means of acetic acid
precipitation of polyhydroxy alkanoates
where the dried biomass is subjected to a temperature between 80°C and the lower limit of the melting range of the polyhydroxy alkanoates over a period of 100 to 120 minutes, and the extracting agent contains 1 to 10 percent by volume of acetic anhydride or β-butyrolactone, relative to the total volume of extracting agent.

## Revendications

1. Procédé de production de polyhydroxyalcanoates à partir d'une biomasse bactérienne, de préférence à partir de la culture de microorganismes methylobacterium rhodesianum IMET 11401 - DSM 8166 MB 126 avec les parties de procédé
séchage de la biomasse humide
extraction de la biomasse séchée avec de l'acide acétique et
précipitation des polyhydroxyalcanoates
caractérisé par le fait que la biomasse bactérienne séchée est exposée dans un espace de temps de 10 à 120 minutes à une température entre 80 °C et la limite inférieure du domaine de fusion des polyhydroxyalcanoates et que l'agent d'extraction contient de 1 à 10 parts en volume en % d'anhydride acétique ou de β-butyrolactone, par rapport au volume total d' agent d'extraction.
